Europäisches Patentamt

**European Patent Office**

Office européen des brevets

(19)

(11) Publication number: **0 092 355**
**A1**

(12) **EUROPEAN PATENT APPLICATION**

(21) Application number: **83302002.7**

(22) Date of filing: **08.04.83**

(51) Int. Cl.³: **C 07 H 15/04**

(30) Priority: **12.04.82 US 367649**

(43) Date of publication of application: **26.10.83**
**Bulletin 83/43**

(84) Designated Contracting States: **BE CH DE FR GB IT LI NL SE**

(71) Applicant: **A.E. STALEY MANUFACTURING COMPANY, 2200 Eldorado Street, Decatur, Illinois 62525 (US)**

(72) Inventor: **Van der Burgh, Leonard Fay, R.R.2, Box 22, Bethany Illinois (US)**
Inventor: **Sommer, Stephen Joseph, 293 Marlene Avenue, Decatur Illinois (US)**

(74) Representative: **Marchant, James Ian et al, Elkington and Fife High Holborn House 52/54 High Holborn, London WC1V 6SH (GB)**

(54) **Preparation of a fatty glycoside mixture.**

(57) The present invention provides a simplified process for preparing surface active glycoside mixtures such as the $C_8$–$C_{22}$ alkyl glycosides directly from hydrophilic saccharide-containing compositions by fatty alcohol interchange. The lipophilic fatty alcohol and hydrophilic saccharide-containing compositions are rendered reactively compatible with one another by incorporating into the reaction medium a sufficient amount of nonionic surface active agent (e.g. long-chain organo glycoside) to permit the fatty alcohol interchange. Long chain-alkyl glycoside mixtures can be easily prepared under acid catalysis and elevated temperatures for either dextrose or lower alkyl glycoside mixtures by direct fatty alkyl alcohol interchange by means of reaction medium which contains a sufficient amount of long-chain alkyl glycoside to render these normally incompatible reactants reactively compatible with one another. Relatively small amounts of nonionic surface active reagents (e.g. 0.2 moles for each mole of anhydrous glucose unit) have been found to significantly increase fatty glycoside mixture yields under the processing conditions of this invention.

ACTORUM AG

## Preparation of a Fatty Glycoside Mixture

This invention relates to the preparation of a fatty glycoside mixture.

The prior art has proposed numerous methods for preparing higher alkyl glucoside mixtures. The most commonly reported technique for preparing such higher alkyl glucoside mixtures involves initially preparing methyl glucoside, converting the methyl glucosides to butyl glucosides with butanol and then converting the butyl group into a fatty alkyl glucoside mixture with a fatty alcohol. United States Patent No. 3,839,318 (Mansfield) discloses a process for directly preparing higher alkyl glucosides from glucose and higher alcohol mixtures. For each mole of glucose reacted with one mole of fatty alcohol, one mole of water is formed. Mansfield indicates that the water should be removed from the reaction medium as fast as it is formed by vacuum distillation or azeotropic distillation. According to Mansfield, oligomerization of fatty alkyl mono-glucosides can be regulated via catalyst concentration and the reaction temperature.

Fatty alkyl esters have been prepared by reacting polyhydric alcohols with fatty acids. United States Patent Nos. 3,644,333 and 3,480,616 (both Osipow et al.) disclose the preparation of glyceryl, sucrose and raffinose fatty esters. The Osipow et al. esterification process involves initially forming an aqueous transparent emulsion of water-insoluble fatty acid and either water-soluble glyceryl, sucrose or raffinose in which the fatty acid esters from a previous run may be used as a surfactant. Relatively large amounts of fatty ester surfactants (e.g. 10-40%) are typically required for the transparent emulsion formation. The transparent emulsion is then reacted at elevated temperatures for a period of time to form the fatty acid esters with water being distilled from the reaction medium to complete the esterification.

According ot the present invention there is provided a method for preparing a fatty glycoside mixture comprising glycosidic components represented by the structural formula $RO(G)_n$ wherein R, represents an organo group containing at least 8 carbon atoms, G represent a saccharide unit and n is a number having a value of at least 1 by chemically reacting a saccharide-containing composition with at least one lipophilic alcohol having at least 8 carbon atoms in the presence of a catalytically effective amount of an acid catalyst, and recovering at least a portion of the glycoside mixture produce from the reaction medium. The method is characterised in that it is carried out in the presence of a surfactant additive represented by the structural formula: $R_fO(G)_n$ wherein $R_f$ represents a lipophilic organo group containing at least 8 carbon atoms, G represents a saccharide unit (which may be the same as or different from the saccharide unit in the glycosidic component), and n is a number having a value of at least 1, the surfactant additive being present in the reaction medium in an amount sufficient to render the saccharide-containing composition and the lipophilic alcohol reactively compatible with one another. Preferably the group G in the surfactant additive is an aldosidic unit.

The reaction is generally carried out by heating the reaction medium to at least 80°C. for a period of time sufficient to convert the alcohol and the composition into a fatty glycoside mixture and at least a portion of the converted fatty glycoside mixture is recovered from said reaction medium.

Under the present invention it is unnecessary to prepare and conduct a series of alcohol interchanges to obtain the desired fatty glycoside mixture. The reaction medium comprised of the saccharide-containing composition, water-insoluble fatty alcohol, surfactant additive (also referred to herein as a reactant additive) and an acid catalyst permits the conversion of the saccharide-containing composition and fatty alcohol reactants into fatty glycoside mixtures.

The term "saccharide-containing composition" herein is intended to cover compositions which molecularly contain at least one saccharide moiety or unit. Unsubstituted or substituted monosaccharide (e.g. aldose) or polysaccharide (e.g. aldoside) containing sugar alcohol or sugar ether units which are reactive with fatty alcohols to form fatty saccharide mixtures may be used for this purpose. Illustrative monosaccharides include aldoses such as apiose, arabinose, galactose, glucose, lyxose, mannose, gallose, altrose, idose, arabinose, ribose, talose, and xylose, the lower aldose ethers thereof (e.g. methoxy-, ethoxy-, propoxy-, butoxy-), polyoxyalkylene (e.g. polyoxyethylene or, polyoxypropylene) aldose ether derivatives which contain terminal hydroxy or lower alkoxy ($C_1$-$C_4$) groups, and mixtures thereof. Illustrative polysaccharides include disaccharides (e.g. maltose, lactose, sucrose), trisaccharides (e.g. maltotriose) saccharides of 4 or more saccharide units (e.g. hemicellulose, inulin, dextrin, dextran, xylan, starch and hydrolyzed starch, low D.E. corn syrup, the polysaccharide ether derivatives thereof such as mentioned above, and mixtures thereof.

In contrast to the reactant additive, the saccharide-containing composition may be suitably represented by the structural formula $A-O-(G)_x$ wherein "A" represents hydrogen or an organo group of less than 8 carbon atoms, "G" represents a saccharide or glycosidic unit with a value (i.e. "X") of at least one. As previously indicated, the saccharide-containing-composition may comprise a substituted or unsubstituted monosaccharide or polysaccharide component. Advantageously a major portion of the saccharide-containing-composition on a molar basis will be comprised of at glycosidic reactant wherein "A" represents at least one of hydrogen or alkyl of less than 4 carbon atoms (e.g. methyl, ethyl and propyl).

The lower alkyl aldoside (especially the lower-alkyl aldoside or glycoside mixtures) are advantageously used to prepare the long chain aldosides. These lower alkyl aldoside mixtures may be conveniently prepared by reacting an aldose-containing composition (e.g. monosaccharide such as glucose, or polysaccharide such as starch) with a lower molecular weight alcohol at an elevated temperature in the presence of an acid catalyst. A particularly convenient method for preparing aldoside mixtures is disclosed in United States Patent 4329449 by Roth et al. In the preferred embodiments of the invention, the major saccharide-containing molar reactant (preferably at least 75%) comprises dextrose, methyl glycoside or a mixture thereof.

The saccharide containing composition is reacted with a fatty or lipophilic alcohol with at least 8 carbon atoms. Monohydric alcohols of from 8-25 carbon atoms are especially well suited for this purpose. Illustrative monohydric alcohols include the primary or secondary, straight or branched, saturated or unsaturated, alkyl or arylalkyl alcohols, ether alcohols, cyclic alcohols, or heterocyclic alcohols. Exemplary alcohols include those represented by the formula: ROH, wherein "R" represents a hydrocarbyl group, such as octyl alcohol, nonyl alcohol, decyl alcohol, dodecyl alcohol, tridecyl alcohol, tetradecyl alcohol, pentadecyl alcohol, dexadecyl alcohol, heptadecyl alcohol, octadecyl alcohol, eicosyl alcohol, pentacosyl alcohol, oleyl alcohol, phenoxyethanol, phenoxypolyethoxyethanol, 2-methyl,7-ethyl,-4-undecanol, and mixtures thereof. Advantageously employed are the alcohols of a molecular weight ranging from 140-300. Alcohols containing a primary alcohol group of 8 to 18 carbon atoms (especially 10 to 14 carbon atoms) are preferred.

The reactants employed to prepare the long chain aldoside mixtures of this invention are incompatible with one another. The saccharide-containing compositions are hydrophilic and readily disperse into polar solvents. The fatty alcohols are lipophilic. When admixed together these reactants are prone to separate into two distinct and non-reactive phases. A reactant additive which renders the incompatible reactants sufficiently compatible with one another to permit the conversion of the reactants into fatty organo glycoside mixture is added to the reaction medium.

According to the invention the reactant additives include those compositions represented by the structural formula: $R_fO(G)_n$ wherein "$R_f$", "G" and "n" respectively represent a lipophilic organo group of at least 8 carbon atoms, an aldosidic unit and a number having a value of at least 1. The $R_f$ moiety encompasses the above mentioned "R" groups. Illustrative "$R_f$" groups include the alkoxy residue of primary or secondary alcohols, straight or branched carbon chains, saturated or unsaturated alkyl or arylalkyl groups, polyoxyalkylene or arylene ether groups, cyclic or heterocyclic organo groups and mixtures thereof.

Fatty alkyl glycosides obtained from the reaction of saccharide-containing compositions with fatty alcohols have been found to be particularly effective agents for converting fatty alcohol and saccharide-containing compositions into fatty alkyl glycoside mixtures. Fractionated or unfractionated fatty glycosides may be used for this purpose. Although a variety of known methods may be used to prepare the reactant additive (e.g. see U.S. Patent Nos. 3,219,656, 3,598,865, 3,640,998, 3,547,828, etc.), fatty glycosides prepared in accordance with this invention are particularly suitable as a reactant additive source. Such fatty glycosides are typically comprised of glycosides having a mixture of varying degrees of glycosidic polymerization (i.e. D.P.) as depicted by the "n" value in the above formula. The fatty glycosidic mixtures prepared by this invention may be fractionated with solvents into fractions of varying oligosaccharide content. Organic solvents, especially the polar solvents, may be effectively applied to fractionate such fatty glycoside mixtures.

Fatty alkyl glycoside mixtures are typically comprised of water-insoluble, acetone-soluble and water-soluble, acetone-insoluble fractions. The acetone soluble fraction will typically contain unreacted or excess fatty alcohol and fatty glycosides of a D.P. 3 or less. The acetone soluble glycosides are predominantely comprised of monoglycoside with di- and tri-glycosides being present in lesser amounts. The acetone-insoluble fraction primarily comprises oligoglycosides of a D.P. 4 or more. Pursuant to this invention the glycosidic reaction product may be fractionated, the desired surfactant recovered with the balance of the reaction product being recycled to the reactor as the $RO/G)_n$ additive. If the desired product constitutes a fatty glycoside mixture of a D.P. 4 or higher, the acetone-insoluble fraction may be recovered by mixing the reaction product with acetone to precipitate the higher oligoglycosides therefrom with the acetone-soluble being recycled to the reactor. If the lower D.P. glycosides are desired, the acetone precipitate may be redispersed into the fatty alcohol feed stream and recycled to the reactor. Alternatively a portion of the fatty glycoside mixture may be recycled to the reactor without fractionation and the balance recovered as the desired product. Under these operating conditions, the R group will typically comprise the same organo moiety as the fatty alcohol reactant. Similar to the fatty alcohol, the

BAD ORIGINAL

R group is preferably an alkyl group of 10 to 14 carbon atoms.

The reaction medium is adjusted to provide a sufficient amount of saccharide-containing composition, fatty alcohol and reactant additive so as to measurably increase the fatty glycoside production in comparison to an interchange reaction conducted without the reactant additive. During the initial stages of the fatty alcohol interchange a sufficient amount of reactant additive to initiate the interaction between the fatty alcohol and saccharide-containing reactant is provided to the reaction media. Thereafter the fatty glycoside mixture produced by the ensuing chemical reaction will increase the total level of fatty glycoside within the reaction medium and permit further conversion of the reactants into the desired fatty glycoside reaction product. In general, effective conversion of the reaction medium into fatty glycoside reaction product will typically be more than 0.005 mole reactant additive for each mole of saccharide-containing composition. Below the 0.1 mole reactant additive level the interchange between the saccharide-containing composition and fatty alcohol proceeds at a considerably slower rate than an initial reaction medium formulated with at least 0.2 mole reactant additive for each anhydrous glucose unit mole. Significantly higher molar concentrations of the reactant additive (e.g. one mole or higher) may be used, if desired, but are not generally required to effectively initiate the interchange and provide a satisfactory conversion rate. The initial charge to the reactor will advantageously provide from 0.2 mole to 0.8 mole reactant additive per anhydrous glucose unit mole. For most operations less than 0.75 mole and preferably from 0.25 mole to 0.5 mole reactant additive for each anhydrous glucose unit mole of reactant is initially charged to the reactor.

0092355

The process lends itself to incremental addition of the saccharide-containing compositions and thereby reduce the total reactant additive requirements for the conversion process. During the initial processing stages, a sufficient amount of reactant additive is added to the reaction medium to permit the fatty glycoside mixture production which, as it is produced, will effectively function as the reactant additive for any further saccharide-containing composition addition to the reaction medium. by this technique a minor proportion of the total saccharide-containing composition requirements may be initially charged to the reactor with reaction product providing the necessary nonionic surface active requirements for reacting the balance of the saccharide-containing composition reactant.

The amount of fatty alcohol provided to the reactor can vary considerably. The fatty alcohol content can also be effectively utilized as a means for controlling the degree of polymerization (D.P.) or glycosidation of the fatty glycoside reaction product. At the lower fatty alcohol concentrations (e.g. 0.5 mole alcohol/anhydrous glucose unit mole), the reaction medium becomes excessively viscous and prone to solidify. The higher fatty alcohol molar concentrations (e.g. 50 mole or higher) tend to unduly dilute the reaction product and excessively form the lower D.P. fatty glycosides. At the lower molar concentrations (e.g. 1 mole) poly-glycoside production is predominantely favored whereas the more elevated concentrations favor formation of the fatty monoglycoside and lower glycoside products. For most operations, the fatty alcohol molar concentration will normally be maintained below the 10 mole level and most typically at about 5 moles for each anhydrous glucose unit mole. For fatty glycoside containing an average degree of polymerization within the D.P. 1 to D.P. 6 range, the reaction will advantageously be conducted within the 1 to 3 fatty alcohol molar range.

The conversion of the saccharide-containing composition to the fatty alkyl glycoside mixture is conducted in the presence of an acidic catalyst at elevated temperatures. Representative acidic catalysts include the strong inorganic and organic acids (e.g. $Pk_a$ less than 3.0 and preferably less than 1.0) and other acid electron accepting compounds such as the Lewis acids (e.g. boron trifluoride, tin tetrachloride, and aluminium chloride, and mixtures thereof. The catalyst concentration, reaction temperature and time, relative proportion of reactants, composition of the saccharide-containing composition and the $RO(G)_n$ additive composition and content may be effectively regulated so as to control the composition of the fatty glycoside mixture.

Illustrative acid catalysts include the strong mineral acids such as hydrochloric, hydrofluoric, hydroidionic, phosphoric, sulfuric, and sulfonic acids, and mixtures thereof. The strong organic acid catalysts include acids such as the phosphonic or sulfonic acid derivatives of alkyl, alkylaryl, aryl, cyclic and heterocyclic organic compounds, and mixtures thereof. Representative strong organic acids include the alkyl phosphonic acids and alkyl sulfonic acids such as the methyl, ethyl, butyl, propyl, amyl, sulfonic or phosphonic acids, phenyl sulfonic acid, phenyl phosphonic acid, paratoluene sulfonic acid, paratoluene phosphonic acid, and mixtures thereof.

If desired, the converting catalyst may comprise a strong acid resin catalyst. Similar to the organic catalysts these resins will normally contain sulfonic and/or phosphonic acid groups which function as the acid converting catalyst. Commercially available sulphonic phenolformaldehyde and polystyrene exchange cationic resins may be effectively used in reactions

0092355

conducted below their thermal decomposition temperatures (e.g. 120°C. or less). Thermally stable resins such as perfluorinated copolymeric resin (e.g. tetra-fluoro-ethylene/perfluoro-3,6-dioxa-4-methyl-7-ocetensulfonic acid commercially available from E.I. du Pont as NAFION-H) (e.g. see C & EN, March 15, 1982, pages 22-25) may also effectively be used and especially in those reactions conducted at temperatures in excess of 110°C.

If desired, the catalyst level may be varied over a broad range. The catalyst level will typically be greater than 0.1 meq catalyst/anhydrous glucose unit mole but less than 100 meq and most typically within the 1-50 meq range. The lower catalyst levels generally apply to those reactions conducted at the higher reaction temperatures and/or for the longer time intervals. Conversely, higher levels may be used in reactions conducted at the lower temperatures and short time intervals. Most appropriately the catalyst concentration will be maintained within the 2 meq to 20 meq and preferably at a level less than 10 meq. It is often desirable to neutralize or remove the catalyst from the fatty glycoside product. The presence of free acid catalyst in the reaction product can lead to compositional alterations in the product, especially when the ultimate usage involves high temperature applications or formulation with chemically reactive additives. Excessive salt contamination arising from neutralizing the free acid catalyst with a base is often unsatisfactory for certain end-use applications (e.g. emulsion polymerization). For these and other reasons it is generally advantageous to empoly in those reactions catalyzed with water-soluble or free acids from 3 to 10 meq and preferably from 4 to 8 meq acid catalyst.

The process is conducted at a temperature and for a period of time sufficient to convert the reactants into the desired glycoside mixture. The reaction will be typically conducted at a temperature greater than 80°C. with most conversions generally falling within the 85°C. to 200°C. range. The reaction time interval can vary considerably depending upon the type of catalyst, catalyst concentration, reactor conditions and type, temperature, and reaction temperature. Although reaction times ranging from as little as a minute or less to 10 hours or more may be adapted to the conversion process, batch-type reactions can be typically completed within 2 to 8 hours with continuous-type reactors being generally conducted within a considerably shorter time interval (e.g. 1 to 20 minutes).

The reaction rate proceeds slowly at temperatures below 90°C. whereas temperatures in excess of 175°C. tend to lead to the formation of colored bodies. Although for certain commercial applications such colored bodies may be acceptable, consumers often find such excessive discoloration to be undesirable. For most commercial operations, the reaction is advantageously conducted at temperatures ranging from 95°C. to 150°C. with operational temperatures ranging from 100°C. to 130°C. being particularly effective for most conversion processes. Within these preferred operational temperatures the conversion process will normally be conducted within 3 to 6 hours in a batch operation. The conversion reaction can be conveniently terminated by cooling the reaction mixture (e.g. below 90°C. such as from 75°C. to 85°C.) or by adding a sufficient amount of base to neutralize the acid catalyst.

A variety of reactors may be used for the conversion process. Continuous or batch-type reactors suitably adapted to the conversion process may be used for this purpose. Reactors operated under a vacuum, atmospheric, superatmospheric pressures or combinations thereof may be used. An illustrative continuous, pressurized reactor system is disclosed in U.S. Patent No. 4,223,129 (Roth et al). Similarly other reactors such as distilling or refluxing reactor systems, pressurized batch-type reactors, continuous wiped film evaporators, scraped heat-exchanges, or plate evaporators may be suitably modified for effective use in the conversion process. Preferably the reaction is conducted at elevated temperatures under a vacuum to permit removal of the more volatile reaction by-products (e.g. water, or lower molecular weight alcohols) from the reaction medium. These more highly volatile constituents may, if desired, be removed at their rate of formation through vacuum distillation.

A major advantage of the present invention is that it provides a simplified method for preparing fatty glycoside mixtures. High quality fatty glycosides can be expeditiously prepared without necessitating the preparation of an intermediate alkyl glycoside such as butyl glycoside. Degradative by-products formation is substantially reduced by eliminating the number of required processing steps and total time interval at elevated temperatures for the overall process. Glycosidation can be more effectively controlled by reducing the extent to which the reactants are subjected to elevated processing temperatures.

The process provides an expeditious and effective direct method for producing fatty glycoside mixtures represented by the structural formula $RO(G)_n$ wherein "R", "G" and "n" represent groups and a value as defined hereinbefore. The fatty group imparts lipophilic properties to the surfactant composition. Hyrophilicity is imparted to the composition by the saccharide moieties of the molecule and, if used, any hydrophilic group (ether linkages) which may be present in the fatty "R" group. The fatty glycoside mixture may be recovered in the form as prepared or, if desired, subjected to further refinement. The hydrophilic and lipophilic balance (HLB) can

BAD ORIGINAL

be controlled by the extent of fatty alcohol derivatization and glycosidation. Hydrophilicity can be achieved by conducting the conversion process so as to favor more extensive polymerization of the glycosidic units. Conversely, lipophilic predominance may be accomplished by increasing the fatty alcohol derivative content and reducing the oligoglycoside content. As a general rule, excess in the molar concentration of lipophilic alcohol will decrease glycosidic unit polymerization with increases in the saccharide reactant molar concentration favoring the production of the more hydrophobic monoglycoside.

The conversion process will generate reaction by-products (e.g. water and/or lower alcohol residues). The predominant by-product produced by conversion will primarily depend upon the moiety interchanged with the fatty alcohol. In a commercial operation it may be advantageous to remove these non-surfactant substances from the reaction product during or after the interchange. Post removal or separation of the low boiling volatiles from the reaction solids and higher boiling such as the fatty alcohols, may be accomplished by conventional techniques such as distillation, flash-cooling or solvent separation.

Any excess or unreacted fatty alcohol is advantageously recycled for reuse in the reaction medium. Such unreacted fatty alcohols may be recovered in a relatively pure form or along with the fatty glycoside portion which is recycled as the reactant additive. Fresh alcohol, saccharide and additional catalyst to compensate for those depleted from the previous run may be appropriately added to the recycle stream to provide a reaction media suitable for use in the ensuing conversion run.

Although the reaction conditions may be controlled so as to promote the formation of either mono- or oligoglycosides, fatty glycoside mixtures of varying degrees of glycosidation are obtained. The fatty glycoside mixture may, if desired, be fractionated into divergent fatty glycoside fractions of differing HLB values. These divergent fractions may, if desired, be recombined to tailor-make a fatty glycoside mixture of a predetermined HLB value. The fractionation may also be adapted so as to permit the recovery of the desired fatty glycoside portion from the mixture with the balance of the materials being recycled to the reactor for the conversion process. Similar to most chemical reactions, it is believed the conversion process provides an equilibrated fatty glycoside mixture. By recycling a portion of the glycosidic reaction product, it is believed that chemical reaction is driven towards the formation of reaction products other than the recycled glycoside. Using this technique, it is believed recycling may be effectively used to enhance yields and efficacy of the conversion process in producing the desired end-product.

The invention is illustrated by the following examples.

## EXAMPLE 1

A dodecyl glycoside mixture was prepared by reacting methyl glucoside mixture, dodecyl alcohol and dodecyl glycoside mixture in a rotary evaporator. The reaction, for 4.5 hours, was conducted in the presence of 6 meq paratoluene sulfonic acid per mole anhydrous glucose unit at 110-120°C. under 0.00-0.03 bars (29-30 inches mercury vacuum). The methyl glucoside mixture used in this example was prepared in accordance with Example 8 of U.S. Patent 4,329,449.Roth et. al. The methyl glucoside mixture obtained from the reactor was allowed to stand for 3 days to effectuate crystallization of a portion of the methyl alpha-D-glucopyranoside therefrom and then filtered through Whatman #2 filter paper. The resultant filtrate, having substantially the same composition as the initial run recycled mother liquor of Table VIII was then used as the methyl glycoside source material in this example. Two-thirds of the total methyl glycoside mixture used to prepare the dodecyl mixture was initially charged to the reactor with the remaining balance being charged to the reactor two hours after initiating the reaction.

The butyl glycoside mixture used to prepare the reactant additive consisted of 34.52% butyl-alpha-monoglycoside, 18.38% butyl-beta-monoglycoside, 25.86% butyl diglycoside, 6.16% butyl triglycoside, 2.21% D.P.$_4$ glycosides and higher, 9.81% dextrose and 3.06% other solid components. Volatiles were stripped from the butyl glycoside by evaporating at 65.6-101.7°C (150-215°F.) under 0.13-0.31 bars (21-26 inches of mercury vacuum). The reaction medium for the fatty alcohol interchange consisted of 426 parts by weight butyl glycoside, 420 parts by weight dodecanol and 2 parts by weight paratoluene sulphonic acid catalyst. The interchange reaction was conducted for 5 hours at 82.2-110°C (180-230°F.) under 0.2-0.44 bars (17-24 inches mercury vacuum). The acetone-insolubles were precipitated from the acetone-solubles by adding, with agitation, 1200 parts, by weight acetone for each 500 parts by weight of the reaction mixture with the admixture being then allowed to quiescently remain for 16 hours. The insolubles were then separated by centrifugation with the soluble portion being recovered and used as the reactant additive in this example. By molecular still distillation of dodecyl alcohol and gas chromatography analysis of the trimethylsilyl derivative it was determined that the acetone-soluble fraction compositionally consisted of 70% dodecyl alcohol, 15.9% dodecyl monoglycoside, 3.4% dodecyl diglycoside, 1% dodecyl triglycoside, 3% butyl glycoside, 0.8% dextrose, 4% higher oligosaccharide and 2% by weight unidentifiable components.

For comparative purposes another run (Run B) was conducted in an identical manner as Run A except for the omission of the dodecyl glycoside additive. In Run B an equivalent amount of dodecyl alcohol was also initially charged to the reactor. The amounts, molar concentrations added to the reactor and compositional analysis of the reaction products for Runs A and B are tabulated below.

TABLE I

| AMOUNTS | RUN A | RUN B |
|---|---|---|
| Acetone-solubles | 66.3g | 0.0 |
| dodecyl alcohol | 46.4g | 46.6g |
| acetone soluble solids | 19.7g | 0.0 |
| | | |
| Methyl glycoside admixture | | |
| initial charge | 7.57g | 16.2g |
| delayed charge | 3.78g | 8.1g |
| | | |
| Molar Concentration | | |
| A-O-(G)$_x$ (Total) | 0.122 mole | 0.125 mole |
| dodecyl glycoside | 0.0375 mole | 0 |
| dodecyl alcohol | 0.25 mole | 0.25 mole |
| methyl glycoside | 0.059 | 0.125 |
| | | |
| Molar ratio dodecyl alcohol: glucose units | 2.16 | 2.0 |
| | | |
| Product Yield | | |
| total solids | 65.9g | 58.9g |
| Acetone-insolubles | 23.8g (36%) | 18.0g (30.6%) |
| bound methanol | 0.05% | 6.33% |
| bound butanol | 0.0% | 0.0% |
| bound dodecyl alcohol | 13.3% | 2.61% |
| Acetone-solubles | 42.1g (64%) | 40.9g (69.4%) |

The total molar concentration of saccharide-containing reactant reacted with the dodecyl alcohol herein were determined upon the basis of the total amount of ingredients charged to the reactor which fulfilled the structural representation A-O-(G)$_x$ wherein "A" represents

either hydrogen or organic moiety of less than eight carbon atoms (e.g. methyl and butyl), "G" a glycosidic unit and "x" the number of glycosidic units in the composition. Saccharide-containing reactants fulfilling this definition and used to prepare the dodecyl glycoside mixture under this example include butyl glycoside (mono- and polyglycosides), dextrose, oligosaccharides and the mono- and polyglycosides of the methyl glycoside mixture and acetone-soluble dodecyl glycoside mixture. The dodecyl alcohol requirement in Run A was met by dodecyl alcohol present in the acetone-solubles.

On a one mole of anhydrous glucose unit basis, 2.05 moles dodecyl alcohol and about 0.31 mole dodecyl glycoside were used to prepare the reaction product in Run A. The initial charge to the reactor contained about 0.36 mole dodecyl glycoside for each mole saccharide-containing composition.

As illustrated by the comparative bound dodecyl alcohol results of Runs A and B, Run A was considerably more effective in converting the saccharide-containing composition and dodecyl alcohol into the desired dodecyl glycoside mixture. The total amount of bound dodecyl alcohol in the Run A product exceeds Run B by more than 5 fold. As illustrated by the comparative analysis, the Run A product contained only 0.05% bound methanol while the Run B product 6.33% or about 126 fold methylation reduction.

The dodecyl glycoside mixture preparation of this example is especially adapted to a process in which at least a portion of acetone solubles (i.e. dodecyl glycoside additive and unreacted dodecyl alcohol) are recycled to the reactor for reuse to produce additional dodecyl glycoside product. The acetone-soluble fraction under these conditions will typically contain about 70% dodecyl alcohol and about 30% by weight acetone-soluble solids. Other reported methods of preparing dodecyl glycoside mixtures can be effectively used as the reactant-additive source material for the initial start-up of the reactor.

EXAMPLE 2

In this example, dodecyl glycosides were prepared using the same apparatus and the processing conditions of Example 1, except for replacing the methyl glycoside recycle mixture of Example 1 with dextrose and the total dextrose amount was initially charged to the reactor. In Run C, the acetone-soluble mixture of Run A was employed to render the hydrophilic reactants compatible with the lipophilic reactants. Run D was conducted under the same conditions except for the omission of the dodecyl glycoside mxiture. The amount and molar concentration used to prepare the reaction product, yields and analysis for the dodecyl glycoside reaction products are tabulated below.

TABLE II

| REACTOR INGREDIENTS | RUN C(g) | RUN D(g) |
|---|---|---|
| acetone-soluble solids | 17.7g | 0.0 |
| dodecyl alcohol | 41.2g | 49.6 |
| glucose hydrate | 11.6 | 26.25 |
| Molar concentrations | | |
| dodecyl glycoside | 0.032 mole | 0.0 |
| $A-O-(G)_x$ [1] | 0.117 mole | 0.133 mole |
| dodecyl alcohol | 0.221 mole | 0.266 mole |
| Yields | | |
| acetone-soluble fraction | 48.2 g (72%) | 49.0 (69.3%) |
| acetone-insoluble | 18.7g (28%) | 21.7 (30.7%) |
| dodecyl alcohol bound | 1.50% | 8.9% |
| butyl alcohol bound | 1.0% | 0.0% |

1- Run C includes the added dextrose as well as the dextrose, oligosaccharides and methyl and butyl glycosides residues of the dodecyl glycoside additive initially charged to the reactor.

On a basis of one mole A-O(G)$_x$, about 0.27 mole dodecyl glycoside and about 1.3 mole added dodecyl alcohol were used to prepare the dodecyl glycoside mixture of Run C. In Run D, 2 moles dodecyl alcohol for each mole of saccharide-containing composition were employed. It was observed that a substantial portion of the dextrose remained as a dextrose precipitate during the Run C and D reactions. Similar to the Run A results, the dodecyl glycoside addition to the reactor (Run C) significantly increased the bound dodecyl alcohol (i.e. dodecyl glycoside product) yields.

0092355

## CLAIMS

1. A method for preparing a fatty glycoside mixture comprising glycosidic components represented by the structural formula $RO(G)_n$ wherein R represents an organo group containing at least 8 carbon atoms, G represents a saccharide unit and n is a number having a value of at least 1, by chemically reacting a saccharide containing composition with at least one lipophilic alcohol having at least 8 carbon atoms in the presence of a catalytically effective amount of an acid catalyst and recovering at least a portion of the glycoside mixture produced from the reaction medium, characterised in that the reaction is carried out in the presence of a surfactant additive represented by the structural formula: $R_fO(G)_n$ where $R_f$ represents a lipophilic organo group containing at least 8 carbon atoms, G represents a saccharide unit (which may be the same as or different from the saccharide unit in the glycosidic component) and n is a number having a value of at least 1, the surfactant additive being present in the reaction medium in an amount sufficient to render the saccharide-containing composition and the lipophilic alcohol reactively compatible with one another.

2. A method as claimed in claim 1, characterised in that the group G in the surfactant additive represents an aldosidic unit.

3. A method as claimed in claim 1 or 2, characterised in that the saccharide-containing composition consists essentially of lower alkyl aldoside.

4. A method as claimed in claim 1 or 2, characterised in that the saccharide-containing composition consists essentially of dextrose.

5. A method as claimed in any of claims 1 to 4 , characterised in that the group $R_f$ is an alkyl group having from 10 to 18 carbon atoms and the surfactant additive is present in a molar concentration is at least 0.2 mole per mole of anhydrous saccharide unit.

6. A method as claimed in any of claims 1 to 5, characterised in that the saccharide-containing composition comprises a composition represented by the structural formula $A-O-(G)_x$ wherein A represents hydrogen or an organo group with less than 8 carbon atoms, G represents a saccharide and x is a number having a value of at least 1.

7. A method as claimed claim 6, characterised in that the lipophilic alcohol is an alkanol of 10 to 18 carbon atoms, the group $R_f$ is an alkyl group of 10 to 18 carbon atoms, G represents a glucosidic unit and A is hydrogen.

8. A method as claimed in claim 7, characterised in that the surfactant additive is present in a molar concentration from 0.25 mole to 0.5 mole per mole of anhydrous glucosidic unit.

9. A method as claimed in claim 5, characterised in that G represents a glucoside unit and the reaction medium contains from 1 mole to 3 mole alkanol and from 0.2 mole to 0.75 mole surfactant additive per mole anhydrous glucose unit.

10. A method as claimed in claim 9, characterised in that the saccharide containing composition is dextrose, or a lower alkyl aldoside having less than 3 alkyl carbon atoms such as a methyl glycoside mixture.

11. A method as claimed in any of claims 1 to 10, characterised in that the fatty glycoside mixture is

0092355

separated into an acetone-soluble fraction and an acetone-insoluble fraction with at least a portion of one of the separated fractions being recycled to the reactor as the surfactant additive.

12.     A method as claimed in claim 11, characterised in that the recycled surfactant additive is the acetone-soluble fraction.

0092355

European Patent Office

**EUROPEAN SEARCH REPORT**

Application number

EP 83 30 2002

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y,D | US-A-3 839 318 (R.C. MANSFIELD) <br> * Claims 8-10 * <br> --- | 1 | C 07 H 15/04 |
| Y | FR-A-2 221 436 (TATE & LYLE) <br> * Page 25; claims 1,6-8 * <br> ----- | 1 | |
| | | | **TECHNICAL FIELDS SEARCHED (Int. Cl. 3)** <br> C 07 H 15/00 |

The present search report has been drawn up for all claims

| Place of search <br> THE HAGUE | Date of completion of the search <br> 02-06-1983 | Examiner <br> VERHULST W. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82